(19) Europäisches Patentamt
European Patent Office
Office européen des brevets

(11) Veröffentlichungsnummer: **0 217 199**
**A1**

(12) # EUROPÄISCHE PATENTANMELDUNG

(21) Anmeldenummer: 86112632.4

(22) Anmeldetag: 12.09.86

(51) Int. Cl.⁴: **C 07 D 233/58**
C 07 D 249/08, A 61 K 31/41-5
A 61 K 31/41, A 01 N 43/50
A 01 N 43/653

(30) Priorität: 23.09.85 DE 3533823

(43) Veröffentlichungstag der Anmeldung: 08.04.87 Patentblatt 87/15

(84) Benannte Vertragsstaaten: AT BE CH DE FR GB IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Siegel, Herbert, Dr.**
**Am Alten Birnbaum 10a**
**D-6238 Hofheim am Taunus(DE)**

(72) Erfinder: **Dittmar, Walter, Dr.**
**Falkensteinstrasse 6**
**D-6072 Dreieich(DE)**

(72) Erfinder: **Dittmar, Walter, Dr.**
**Uhlandstrasse 10**
**D-6238 Hofheim am Taunus(DE)**

(54) **Alkenylazole, Verfahren zu ihrer Herstellung und ihre Verwendung.**

(57) Alkenylazole der Formel I a/b

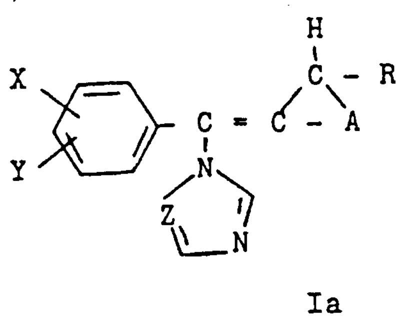

Ia

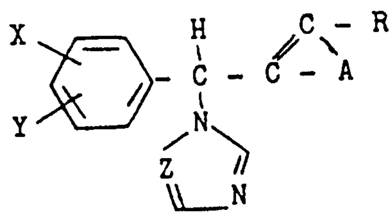

Ib

mit X/Y gleich H, Alkyl, Phenyl, Hal, Alkoxy, Alkylthio; Z gleich CH oder N; R gleich Alkyl oder eine überbrückte $(CH_2)_m$-Kette; A gleich Alkylen oder eine überbrückte Alkylenkette; sowie die Salze dieser Alkenylazole sind Antimykotika bzw. Fungizide. Sie werden erhalten durch Wasserabspaltung aus den Verbindungen II

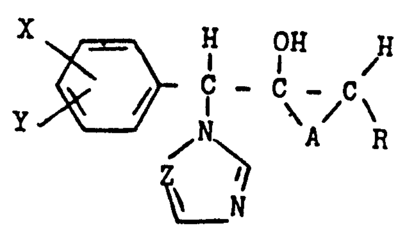

II

EP 0 217 199 A1

Croydon Printing Company Ltd

# Alkenylazole, Verfahren zu ihrer Herstellung und ihre Verwendung

Die Erfindung betrifft Alkenylazole, Verfahren zu ihrer Herstellung und ihre Verwendung als Antimykotica und Fungizide.

Aus den Offenlegungsschriften EP-OS 57 365 und DE-OS 2.652.313 ist bekannt, daß Alkenylazole zur Bekämpfung von Pilzen bei Mensch, Tier und Pflanze geeignet sind. Die Wirksamkeit dieser Verbindungen ist jedoch insbesondere bei niedrigen Konzentrationen nicht befriedigend.

Die Erfindung betrifft daher Alkenylazole der allgemeinen Formel Ia/Ib

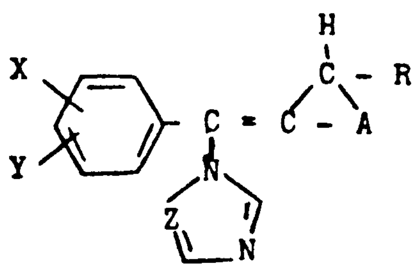

Ia

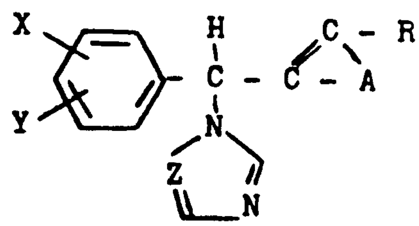

Ib

in welcher

X und Y gleich oder verschieden sind und H, $(C_1-C_4)$-Alkyl, Phenyl, F, Cl, Br, $(C_1-C_4)$-Alkoxy oder $(C_1-C_4)$-Alkylthio bedeuten

Z CH oder N bedeutet

R H oder $(C_1-C_4)$-Alkyl oder, wenn A eine überbrückte $(CH_2)_m$-Kette mit $m = 4$ und 3 Brückenkohlenstoffatomen ist, eine mit dem mittleren Brückenkohlenstoffatom verbundene Methylengruppe bedeutet,

A eine $(CH_2)_n$-Kette mit $n = 1 - 10$, die unsubstituiert oder mit $(C_1-C_4)$-Alkyl sowie Phenyl substituiert ist,
oder
eine überbrückte $(CH_2)_m$-Kette mit $m = 3$ oder 4 und 1 - 5 Brückenkohlenstoffatomen bedeutet

sowie ihre Salze mit Säuren und ihre Stereoisomeren. Die beiden Formeln Ia und Ib zeigen entweder eine exocyclische oder eine endocyclische C=C-Doppelbindung.

Von den Verbindungen der Formel Ia/Ib sind solche bevorzugt, bei denen X und Y Wasserstoff, Fluor, Chlor oder Phenyl sind, R = H oder $CH_2$ ist und A eine $(CH_2)_n$-Kette mit n = 3 - 5 oder eine überbrückte $(CH_2)_m$-Kette mit m = 3 oder 4 und 1 - 5 Brückenkohlenstoffatomen bedeutet, wobei wenigstens eines dieser Merkmale erfüllt sein muß.
Die Herstellung der erfindungsgemäßen Verbindungen der Formel Ia/Ib erfolgt durch Abspaltung von Wasser aus Carbinolen der Formel II

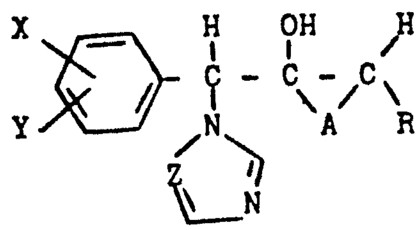

II

worin X, Y, Z, R und A die zu Formel I angegebene Bedeutung haben.

Die Wasserabspaltung aus Verbindungen der Formel II kann prinzipiell nach in der Literatur beschriebenen Methoden erfolgen. Normalerweise arbeitet man in flüssiger Phase unter Verwendung saurer Dehydratisierungsmittel bei Temperaturen zwischen 0° und 150°C, vorzugsweise bei 25 - 100°C. Als saure Dehydratisierungsmittel kommen anorganische und organiscche Protonsäuren wie Schwefelsäure, Essigsäure oder p-Toluolsulfonsäure sowie deren Anhydride und Säurechloride, saure Salze, wie $KHSO_4$, oder Lewis Säuren, wie z.B. Borfluorid, Zinkchlorid, infrage; bevorzugt verwendet man jedoch eine Protonsäure wie Schwefelsäure. Das Dehydratisierungsmittel kann gleichzeitig als Solvens dienen, man kann jedoch auch gegen-

über dem Dehydratisierungsmittel inerte andere Lösungsmittel wie Tetrahydrofuran oder Toluol verwenden. Die Aufarbeitung erfolgt durch Neutralisation mit wäßriger Lauge, wobei sich das Reaktionsprodukt entweder direkt abscheidet oder mit einem Lösungsmittel extrahiert wird. Zur Reinigung kristallisiert man die Produkte entweder um oder chromatographiert an Kieselgel.

Die als Vorstufe verwendeten Carbinole der allgemeinen Formel II können auf zwei Wegen erhalten werden wie in der parallelen deutschen Patentanmeldung P 35 33 824.5 (HOE 85/F202 ) beschrieben ist.

Bei der ersten Methode versetzt man ein Benzylazol der allgemeinen Formel III,

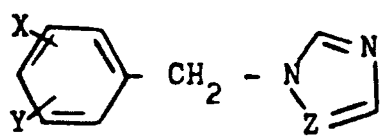

III

in welcher X, Y und Z die zu Formel I angegebene Bedeutung haben, z.B. in einem aprotischen polaren Lösungsmittel bei einer Temperatur zwischen 25° und -100°C mit einem oder zwei Äquivalenten einer starken Base, gibt zu dem entstandenen Carbanion eine Carbonylverbindung der allgemeinen Formel IV,

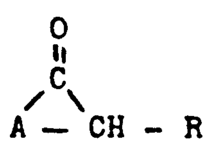

IV

in welcher A und R die zu Formel I angegebene Bedeutung haben, und erhält schließlich nach Zugabe einer Protonsäure das Carbinol der allgemeinen Formel II.

Als starke Basen kommen vor allem Akalimetallhydride, wie Natriumhydrid, oder Erdalkali- und Alkalimetallalkyle, wie z.B. Butyllithium oder metallierte Amine, wie z.B. Lithiumdiisopropylamid, in Frage. Die Verwendung von zwei Äquivalenten starker Base ist z.B. bei Benzyltriazolderivaten notwendig, andernfalls erhält man Produkte, die am Triazolring hydroxyalkyliert sind. Die Reaktion kann in den für metallorganische Reaktionen gebräuchlichen Lösungsmitteln wie Dimethylformamid, Dimethylsulfoxid, Dimethoxyethan, Diethylether oder vorzugsweise Tetrahydrofuran durchgeführt werden. Außerdem können Lösungsmittelzusätze wie N,N,N',N'-Tetramethylethylendiamin oder Hexamethylphosphorsäuretriamid Verwendung finden. Die Umsetzung mit der starken Base und der Carbonylverbindung der Formel III kann bei Temperaturen zwischen Zimmertemperatur und -100°C erfolgen, geschieht jedoch vorzugsweise im Bereich zwischen -30°C und -80°C. Die Verbindungen der Formel II werden in der bei metallorganischen Reaktionen üblichen und bekannten Weise isoliert. Die Reinigung der Verbindungen der Formel II erfolgt in der Regel durch Umkristallisation aus einem organischen Lösungsmittel, wie z.B. Hexan, Cyclohexan, Ethanol, Essigsäureethylester oder durch Säulenchromatographie an Kieselgel.

Die verwendeten N-Benzylazole der Formel III werden nach bekannten Methoden durch Alkylierung von Imidazol, bzw. Triazol mit Benzylhalogeniden erhalten.

Bei der zweiten Methode geht man von Oxiranen der allgemeinen Formel V aus,

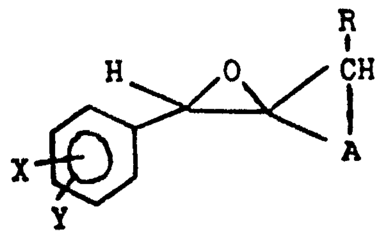

V

worin X, Y, R und A die zu Formel I angegebene Bedeutung besitzen. Die Umsetzung zu Carbinolen der Formel II erfolgt, indem man das Epoxid der Formel V mit einem Azol oder seinem Natriumsalz bei einer Temperatur zwischen 0 und 80°C, vorzugsweise im Bereich zwischen 0 und 50°C in

einem polaren Lösungsmittel öffnet. Oxirane der Formel V erhält man nach literaturbekannten Methoden. Dazu wird zunächst das zugrunde liegende Olefin hergestellt und dieses mit einer Persäure oxidiert.

Aus den Azolderivaten der allgemeinen Formel I können Säureadddditionssalze hergestellt werden. Dazu kommen alle Säuren, die physiologisch verträgliche Salze bilden, infrage. Dazu gehören sowohl anorganische Säuren, wie Chlorwasserstoffsäure, Salpetersäure und Schwefelsäure, als auch mono- und bifunktionelle organische Säuren, insbesondere Carbonsäuren, wie Essigsäure, Bernsteinsäure, Weinsäure oder organische Sulfonsäuren wie p-Toluolsulfonsäure, Benzolsulfonsäure usw.

Die neuen Verbindungen der allgemeinen Formel (I) und ihre Säureadditions-Salze sind wertvolle Arzneimittel. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als geeignete Anwendungsform der erfindungsgemäßen Verbindung kommen beispielsweise in Frage: Tabletten oder Kapseln, Suspensionen, Lösungen, Gelees, Cremes oder Salben sowie Aerosole in Form von Sprays.

Die Anwendungskonzentrationen für Lösungen, Gelees, Cremes oder Salben sowie Aerosole in Form von Spray beträgt im allgemeinen zwischen 0,1 - 20, vorzugsweise 0,5 - 5 Gewichtsprozenten.

Die orale Anwendung erfolgt in pharmazeutisch üblichen Zubereitungen, beispielsweise in Form von Tabletten oder Kapseln, die pro Tagesdosis 20 - 500 mg, vorzugsweise 50 bis 300 mg des Wirkstoffes in Mischung mit einem gebräuchlichen Trägerstoff und/oder Konstituents enthalten.

Für die lokale Anwendung können z.B. Suspensionen, Lösungen, Gelees, Cremes, Salben oder Suppositorien verwendet werden.

Für die parenterale Anwendung kommen geeignete Suspensionen oder Lösungen in Frage in einer Anwendungskonzentration zwischen 0,1 - 5 Gewichtsprozenten.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener phytopathogener Pilze, wie z. B. Piricularia oryzae, Pellicularia sasakii, verschiedene Rostarten, vor allem aber Venturia inaequalis, Cercospora-Arten und Echte Mehltaupilze im Obst-, Gemüse-, Getreide- und Zierpflanzenbau.

Die Verbindungen der Formel I eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z. B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:
Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester,

Polyoxethylen-sorbitan-fettsäureester oder Polyoxethylensorbitester.

Stäubemittel kann man durch Vermahlen des Wirkstoffes mit fein verteilten, festen Stoffen, z. B. Talkum, natürlichen Tonen wie Kaolin, Bentonit, Pyrophyllit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen.
Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich. Insbesondere bei Mischungen mit Fungiziden werden teilweise auch synergistische Wirkungssteigerungen erzielt werden.

Die nachfolgenden Beispiele erläutern die Herstellung der erfindungsgemäßen Verbindungen.

Beispiel 1 (Verbindung 1)

1-Imidazolyl-4-chlorphenyl-2-bicyclo[2.2.1]heptyliden-methan

8,0 g (26,4 mmol) 1-Imidazolyl-(4-chlorphenyl)-(2-hydroxy-2-bicyclo[2.2.1]heptyl)methan wurden in einem Gemisch aus 48 ml Eisessig und 12 ml conc. Schwefelsäure gelöst und 2 Stunden auf 100°C erhitzt. Anschließend wurde abgekühlt und das Reaktionsgemisch unter Kühlen in 200 ml 25 %ige Natronlauge gegeben. Man extrahierte mit Methylenchlorid, trocknete die organische Phase, engte ein und erhielt 6,5 g Rohprodukt. Nach Säulenchromatographie an Kieselgel (Laufmittel Cyclohexan/Ethylacetat = 1 : 1) ergaben sich 4,0 g reines Imidazolderivat als Öl.

$C_{17}H_{17}ClN_2$ (284,80)

| | | | |
|---|---|---|---|
| ber. | C 71,70 | H 6,02 | N 9,84 |
| gef. | C 71,7 | H 5,9 | N 9,6 |

0217199

Beispiel 2 (Verbindung 2)

1-Imidazolyl-(2-chlor-6-fluorphenyl)-1-cyclohexenyl-methan

3,3 g (10,7 mmol) 1-Imidazolyl-(2-chlor-6-fluorphenyl)-1-(1-hydroxy-cyclohexyl)methan wurden in 19,5 ml Eisessig gelöst, anschließend gab man 4,9 ml conc. Schwefelsäure zu, erwärmte 1 Stunde auf 100°C und ließ auf Zimmertemperatur abkühlen. Die Aufarbeitung erfolgte analog Beispiel 1. Durch Säulenchromatographie (Cyclohexan : Ethylacetat= 2 : 1) wurden aus 3,1 g Rohprodukt 2,9 g Imidazolderivat isoliert.

$C_{16}H_{16}ClFN_2$(290,78)

| | | | |
|---|---|---|---|
| ber. | C 66,09 | H 5,55 | N 9,34 |
| gef. | C 65,4 | H 5,6 | N 9,3 |

Beispiel 3 (Verbindung 3)

1-(1.2.4-Triazolyl)-2-chlorphenyl)-(2-adamantyliden)-methan

7,0 g (10,4 mmol) 1-(1,2,4-Triazolyl)-2-chlorphenyl-2-(2-hydroxyadamantyl)methan wurden in einem Gemisch aus 36 ml Eisessig und 9 ml $H_2SO_4$ conc. auf 100° erwärmt und 2 h bei dieser Temperatur gerührt. Nach Abkühlen des Reaktionsgemischs wurde wie in Beispiel 1 aufgearbeitet. Aus 5,8 g Rohprodukt wurden durch Umkristallisation aus Hexan/Isopropanol (1 : 1) 5,0 g reines Triazolderivat erhalten.

$C_{19}J_{20}ClN_3$ (325,85)

| | | | |
|---|---|---|---|
| ber. | C 70,04 | H 6,19 | N 12,89 |
| gef. | C 69,9 | H 6,3 | N 12,5 |

Analog Beispiel 1 - 3 wurden folgende Verbindungen der Formel I hergestellt.

| Bsp. Nr. | 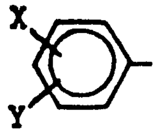 | Doppelbindung exocycl. endocycl. | R | Z | A | Fp.[°C] |
|---|---|---|---|---|---|---|
| 4 | 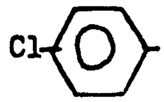 | endo | H | CH | $-(CH_2)_3-$ | Öl |
| 5 | 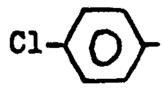 | endo | H | CH | $-(CH_2)_4-$ | Öl |
| 6 | 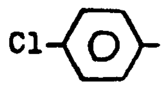 | exo | $-CH_2-$ | CH | 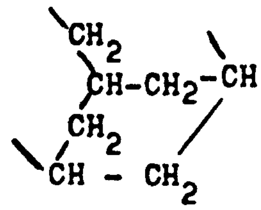 | 103 |
| 7 | 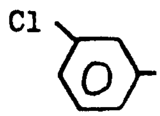 | endo | H | CH | $-(CH_2)_4-$ | Öl |
| 8 | 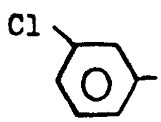 | exo | $-CH_2-$ | CH | 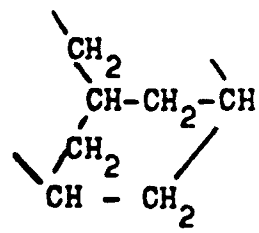 | 108 |
| 9 | 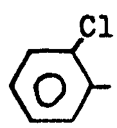 | endo | H | CH | $-(CH_2)_5-$ | Öl |
| 10 | 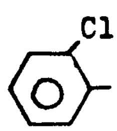 | endo | H | CH | 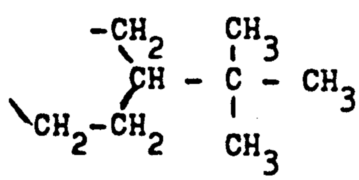 | Öl |
| 11 | 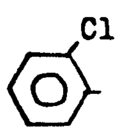 | endo | H | CH | 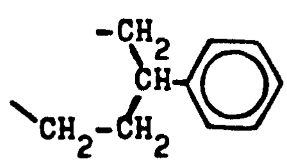 | Öl |
| 12 | 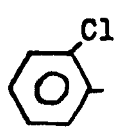 | exo | H | CH | 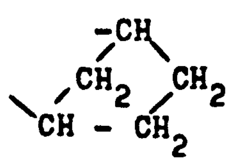 | Öl |

Fortsetzung

| Bsp. Nr. | 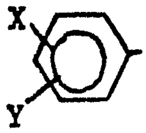 | Doppelbindung exocycl. endocycl. | R | Z | A | Fp.[°C] |
|---|---|---|---|---|---|---|
| 13 | 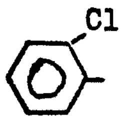 | exo | $-CH_2-$ | CH | 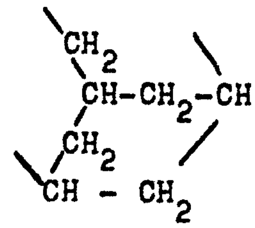 | 125 |
| 14 | 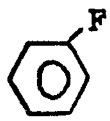 | exo | $-CH_2-$ | CH | 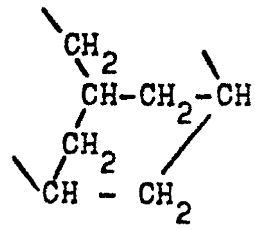 | 117 |
| 15 | 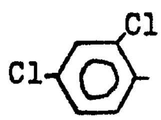 | endo | H | CH | $-(CH_2)_4-$ | Öl |
| 16 | 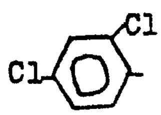 | endo | H | CH | $-(CH_2)_5-$ | Öl |
| 17 | 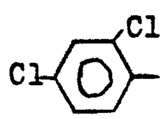 | endo | H | CH | $-(CH_2)_{10}-$ | 133 |
| 18 | 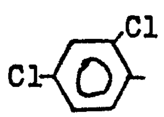 | exo | H | CH | 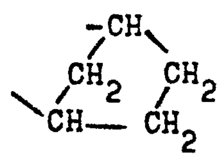 | Öl |
| 19 | 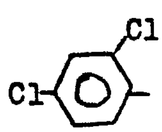 | exo | H | CH | 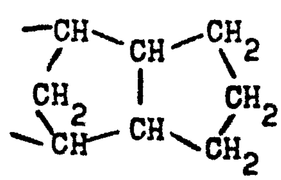 | Öl |

Fortsetzung

| Bsp. Nr. | 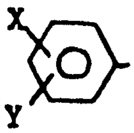 | Doppelbindung exocycl. endocycl. | R | Z | A | Fp. [°C] |
|---|---|---|---|---|---|---|
| 20 | 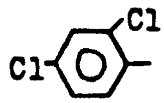 | exo | $-CH_2-$ | CH | 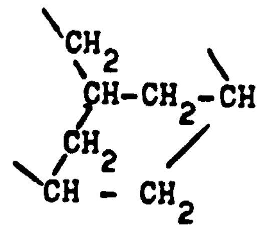 | 148 |
| 21 | 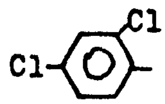 | endo | H | CH | $-(CH_2)_4-$ | 81 |
| 22 | 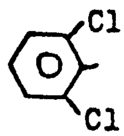 | exo | $-CH_2-$ | CH | 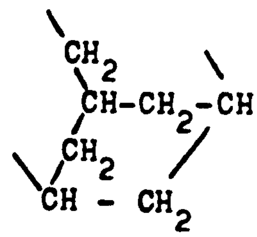 | 114 |
| 23 | 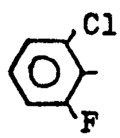 | endo | H | CH | $-(CH_2)_4-$ | 81 |
| 24 | 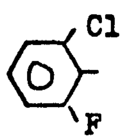 | endo | H | CH | $-(CH_2)_5-$ | 78 |
| 25 | 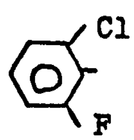 | exo | $-CH_2-$ | CH | 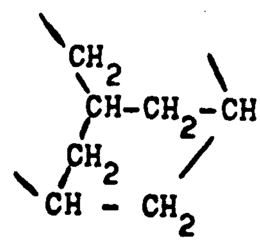 | 120 |
| 26 | 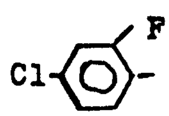 | endo | H | CH | $-(CH_2)_4-$ | 81 |

| Bsp. Nr. | 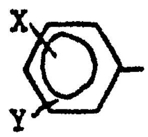 | Doppel-bindung exocycl. endocycl. | R | Z | A | Fp.[°C] |
|---|---|---|---|---|---|---|
| 27 | 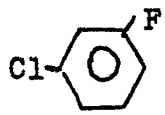 | endo | H | CH | 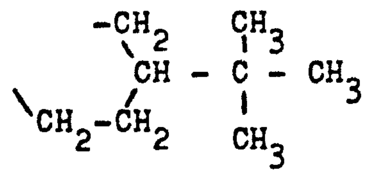 | Öl |
| 28 | 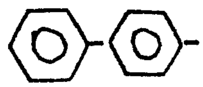 | endo | H | CH | $-(CH_2)_3-$ | Öl |
| 29 | 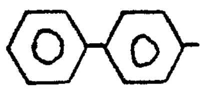 | endo | H | CH | $-(CH_2)_4-$ | Öl |
| 30 | 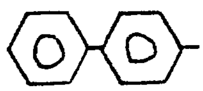 | endo | H | CH | $-(CH_2)_5-$ | Öl |
| 31 | 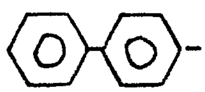 | endo | H | CH | $-(CH_2)_{10}-$ | 97 |
| 32 | 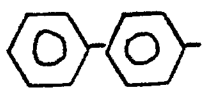 | exo | $CH_2$ | CH | 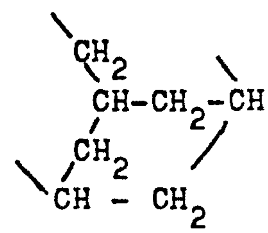 | 177 |
| 33 | 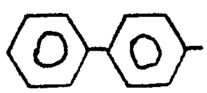 Z-Isomer | exo | H | CH | 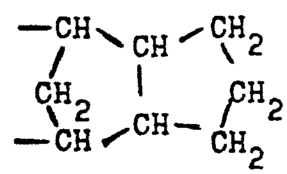 | Öl |
| 34 | 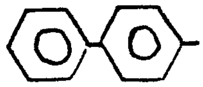 E-Isomer | exo | H | CH | 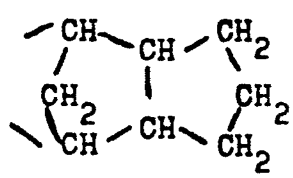 | 142° |

Fortsetzung 

| Bsp. Nr. | 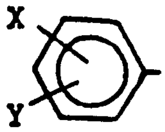 | Doppelbindung exocycl. endocycl. | R | Z | A | Fp. [°C] |
|---|---|---|---|---|---|---|
| 35 | 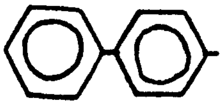 | | H | N | $-(CH_2)_4-$ | Öl |
| 36 | 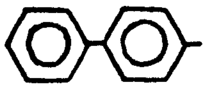 | | $CH_3$ | N | $-(CH_2)_4-$ | Öl |

A) Herstellung des Ausgangsmaterials zu Verbindung 2

1-Imidazolyl-(2-chlor-6-fluorphenyl)-1-(1-hydroxycyclohexyl)methan

10,6 g (50 mmol) (2-Chlor, 6-fluorbenzyl)-1-imidazol wurden in 100 ml Tetrahydrofuran gelöst und bei -70°C unter Stickstoffatmosphäre mit 32 ml (50 mmol) n-Butyllithium in Hexan versetzt. Man rührte eine halbe Stunde nach und gab dann 4,9 g (50 mmol) Cyclohexanon so zu, daß die Temperatur bei -70°C gehalten werden konnte. Innerhalb von drei Stunden wurde das Reaktionsgemisch auf Zimmertemperatur erwärmt, dann mit Wasser versetzt und mit Methylenchlorid extrahiert. Trocknen und Einengen der organischen Phase ergaben 14,8 g rohes Carbinol, das aus Xylol umkristallisiert wurde. Man erhielt 11,6 g weiße Kristalle vom Fp. 170°C.
$C_{16}H_{18}ClFN_2O$ (308,78)

| | | | |
|---|---|---|---|
| ber. | C 62,44 | H 5,57 | N 9,1 |
| gef. | C 62,8 | H 5,9 | N 9,2 |

B) Herstellung des Ausgangsmaterials zu Verbindung 3

1-(1,2,4-Triazolyl)-2-chlorphenyl-2-(2-hydroxy-adamantanyl)methan

Zu einer Lösung von 9,7 g (50 mmol) 1-(2-Chlorbenzyl)-1,2,4-triazol und 6 g (50 mmol) N,N,N',N'-Tetramethylethylendiamin in 100 ml Tetrahydrofuran tropfte man unter Stickstoffatmosphäre bei -78°C 66 ml (100 mmol) n-Butyllithium in Hexan, rührte eine halbe Stunde bei dieser Temperatur nach und gab dann 7,5 g (50 mmol) 2-Adamantanon in Tetrahydrofuran so zu, daß -70°C nicht überschritten wurden. Das Reaktionsgemisch wurde noch 2 Stunden bei -78°C gehalten und dann auf Zimmertemperatur aufwärmen gelassen. Nach Aufarbeitung mit Wasser/Methylenchlorid erhielt man 17,4 g Rohprodukt, das nach Umkristallisation aus Essigsäureethylester 10,0 g Triazolderivat vom Fp. 178°C ergab.
$C_{19}H_{22}ClN_3O$ (343,86)

| | | | |
|---|---|---|---|
| ber. | C 66,37 | H 6,45 | N 12,22 |
| gef. | C 66,8 | H 6,6 | N 11,8 |

C) Herstellung des Ausgangsmaterials zu Verbindung 1

1-Imidazolyl-4-chlorphenyl-2-(2-hydroxybicyclo[2.2.1]-heptyl)methan

Zu einer analog Beispiel 3 hergestellten Lösung von 9,65 g (50 mmol) 1-Imidazolyl-4-chlorphenylmethyllithium in 100 ml Tetrahydrofuran tropfte man bei -70°C langsam 5,5 g (50 mmol) 2-Norbornan in 20 ml Tetrahydrofurn und ließ auf Raumtemperatur aufwärmen. Die Aufarbeitung mit Wasser/Methylenchlorid ergab 9,4 g eines Öls, das nach Umkristallisation mit Cylcohexan/Ethylacetat (1 : 10) 8,1 g Kristalle vom Schmp. 118°C lieferte.

$C_{17}H_{19}ClN_2O$

| | | | |
|---|---|---|---|
| ber. | C 67,44 | H 6,32 | N 8,74 |
| gef. | C 67,9 | H 6,6 | N 8,5 |

Patentansprüche

1. Verbindung der Formel Ia/Ib

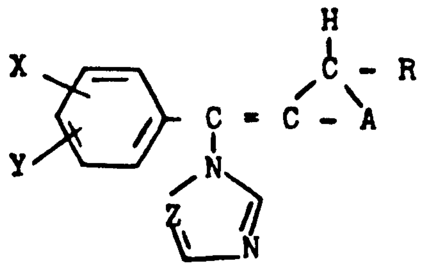

Ia

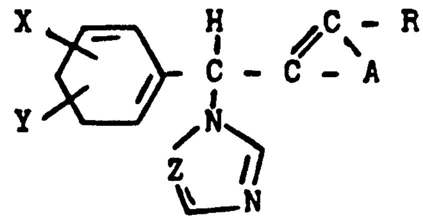

Ib

in welcher

X und Y gleich oder verschieden sind und H, $(C_1-C_4)$-Alkyl, Phenyl, F, Cl, Br, $(C_1-C_4)$-Alkoxy oder $(C_1-C_4)$-Alkylthio bedeuten

Z CH oder N bedeutet

R H oder $(C_1-C_4)$-Alkyl oder, wenn A eine überbrückte $(CH_2)_m$-Kette mit m= 4 und 3 Brückenkohlenstoffatomen ist, eine mit dem mittleren Brückenkohlenstoffatom verbundene Methylengruppe bedeutet,

A eine $(CH_2)_n$-Kette mit n = 1 - 10, die unsubstituiert oder mit $(C_1-C_4)$-Alkyl sowie Phenyl substituiert sein kann,
oder
eine überbrückte $(CH_2)_m$-Kette mit m = 3 oder 4 und 1 - 5 Brückenkohlenstoffatomen bedeutet

sowie ihre Salze mit Säuren und ihre Stereoisomeren.

2. Verbindung der Formel Ia/Ib nach Anspruch 1, dadurch gekennzeichnet, daß mindestens eines der Merkmale

X und Y Wasserstoff, Fluor, Chlor oder Phenyl,

R Wasserstoff oder $CH_2$

A eine $(CH_2)_n$-Kette mit n = 3 - 5 oder
eine überbrückte $(CH_2)_m$-Kette mit m = 3 oder 4 und 1 - 5 Brückenkohlenstoffatomen

bedeutet.

0217199

3. Verfahren zum Herstellen einer Verbindung I nach Anspruch 1, dadurch gekennzeichnet, daß man aus Carbinolen der Formel II

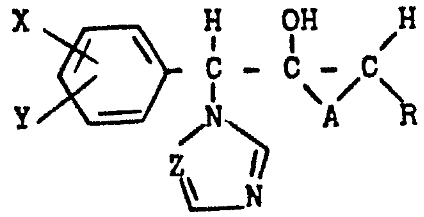

II

worin X, Y, Z, A und R die genannten Bedeutungen haben, Wasser abspaltet.

4. Verwendung einer Verbindung I nach Anspruch 1 als Antimykotikum.

5. Verwendung einer Verbindung I nach Anspruch 1 als fungizides Mittel.

6. Verwendung einer Verbindung I nach Anspruch 1 zur Herstellung eines Antimykotikums.

7. Verwendung einer Verbindung I nach Anspruch 1 zur Herstellung einer fungiziden Zubereitung.

8. Verfahren zum Behandeln von Mykosen, dadurch gekennzeichnet, daß man eine Zubereitung anwendet, die eine Verbindung I nach Anspruch 1 enthält.

Patentansprüche Österreich:

0217199

1. Verfahren zum Herstellen von Verbindungen der Formel Ia/Ib

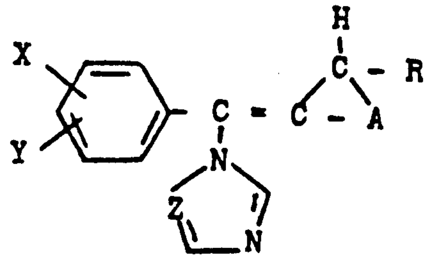

Ia

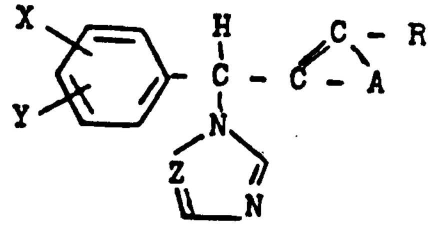

Ib

in welcher

X und Y gleich oder verschieden sind und H, $(C_1-C_4)$-Alkyl, Phenyl, F, Cl, Br, $(C_1-C_4)$-Alkoxy oder $(C_1-C_4)$-Alkylthio bedeuten

Z CH oder N bedeutet

R H oder $(C_1-C_4)$-Alkyl oder, wenn A eine überbrückte $(CH_2)_m$-Kette mit m= 4 und 3 Brückenkohlenstoffatomen ist, eine mit dem mittleren Brückenkohlenstoffatom verbundene Methylengruppe bedeutet,

A eine $(CH_2)_n$-Kette mit n = 1 - 10, die unsubstituiert oder mit $(C_1-C_4)$-Alkyl sowie Phenyl substituiert ist,

oder

eine überbrückte $(CH_2)_m$-Kette mit m = 3 oder 4 und 1 - 5 Brückenkohlenstoffatomen bedeutet

sowie ihrer Salze mit Säuren und ihrer Stereoisomeren, dadurch gekennzeichnet, daß man

a) aus Verbindungen II

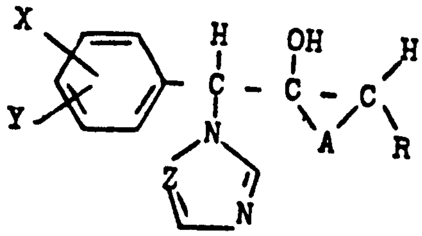

II ,

worin X, Y, Z, R und A die zu Formel I angegebene Bedeutung haben, Wasser abspaltet; oder daß man

b) ein Benzylazol der Formel III,

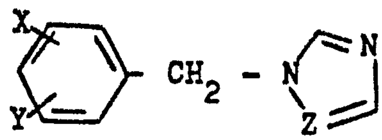

III

in welcher X, Y und Z die zu Formel I angegebene Bedeutung haben, mit einem oder zwei Äquivalenten einer starken Base versetzt, dann zu dem entstandenen Carbanion eine Carbonylverbindung der Formel IV,

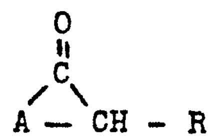

IV

gibt, in welcher A und R die zu Formel I angegebene Bedeutung haben, dann durch Zugabe einer Protonsäure das Carbinol der Formel II herstellt, und daß man aus diesem nach Verfahrensvariante a) Wasser abspaltet; oder daß man

c) Oxirane der Formel V

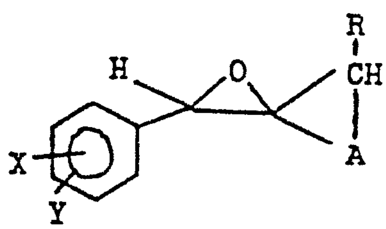

V

worin X, Y, R und A die zu Formel I angegebene Bedeutung besitzen, mit einem Azol der Formel

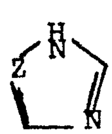

, in der Z die zu Formel I angegebene Bedeutung hat,

oder seinem Natriumsalz, öffnet, und daß man aus der dabei entstehenden Verbindung II, wie in Verfahrensvariante a) angegeben, Wasser abspaltet.

2. Verfahren nach Anspruch 1, dadurch gekennzeichnet, daß mindestens eines der Merkmale

X und Y Wasserstoff, Fluor, Chlor oder Phenyl,

R Wasserstoff oder $CH_2$

A eine $(CH_2)_n$-Kette mit n = 3 - 5 oder eine überbrückte $(CH_2)_m$-Kette mit m = 3 oder 4 und 1 - 5 Brückenkohlenstoffatomen

bedeutet.

3. Verfahren zum Behandeln von Mykosen, dadurch gekennzeichnet, daß man eine Zubereitung andwendet, die eine Verbindung I nach Anspruch 1 enthält.

0217199

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung

EP 86112632.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP - A2 - 0 121 081 (BASF)<br>* Formel I; Zusammenfassung *<br>-- | 1,5-7 | C 07 D 233/58<br>C 07 D 249/08<br>A 61 K 31/415<br>A 61 K 31/41<br>A 01 N 43/50<br>A 01 N 43/653 |
| A | CHEMICAL ABSTRACTS, Band 94, Nr. 15, 13. April 1981, Columbus, Ohio, USA<br>KASHIMA, CHOJI; TAJIMA, TADAKUNI "Preparation of β-(1-imidazolyl)-enones."<br>Seite 688, Spalte 2, Zusammenfassung-Nr. 121405k<br>& Synthesis 1980, (11), 880-1<br>-- | 1 | |

RECHERCHIERTE SACHGEBIETE (Int Cl 4)

C 07 D 233/00

C 07 D 249/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5-7

Unvollständig recherchierte Patentansprüche: –

Nicht recherchierte Patentansprüche: 4,8

Grund für die Beschränkung der Recherche:

(Art. 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 23-12-1986 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 08.82

Europäisches Patentamt

# EUROPÄISCHER TEILRECHERCHENBERICHT

Nummer der Anmeldung

EP 86112632.4

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, Band 101, Nr. 7, 13. August 1984, Columbus, Ohio, USA<br>KASHIMA, CHOJI; TAJIMA, TADAKUNI; OMOTE, YOSHIMORI. "The compounds related to 3-(imidazolyl)-2-alken-1-ones. Preparation and reactions." Seite 614, Spalte 2, Zusammenfassung-Nr. 54998d<br>& J. Heterocycl Chem. 1984, 21(1), 171-5<br>-- | 1 | |
| A | DE - A1 - 2 738 640 (JCJ)<br>* Anspruch 1; Beispiele 76, 129 *<br>---- | 1 | RECHERCHIERTE SACHGEBIETE (Int Cl 4) |